# EUROPEAN PATENT SPECIFICATION

(11) **EP 0 934 240 B1**
(45) Date of publication and mention of the grant of the patent: **10.01.2001**
(21) Application number: 96933786.4
(22) Date of filing: 13.09.1996
(51) Int. Cl.: C07C 37/00, C07C 39/14, C07C 43/225, C07C 49/255, C07C 59/64

(54) **PRODUCTION OF 6-BROMO-2-NAPHTHOL AND DERIVATIVES**
HERSTELLUNG VON 6-BROM-2-NAPHTHOL UND DERIVATEN
PRODUCTION DE 6-BROMO-2-NAPHTOL ET DE SES DERIVES

(43) Date of publication of application: 11.08.1999
(73) Proprietor: ALBEMARLE CORPORATION, Baton Rouge Louisiana 70801 (US)
(72) Inventor: SABAHI, Mahmood, Baton Rouge, LA 70810 (US)
(74) Representative: UEXKÜLL & STOLBERG
(86) International application number: US9614777
(87) International publication number: WO9811041

(56) References cited:
- GB-A- 380 563
- US-A- 5 225 603
- US-A- 5 536 870

## Description

This invention relates to processes for preparing 6-bromo-2-naphthol and derivatives thereof from 1,6-dibromo-2-naphthol.

### Background

6-Bromo-2-naphthol is a compound which has particular value as an intermediate in the preparation of non-steroidal antiinflammatory agents, such as 4-(6-methoxy-2-naphthyl)-2-butanone (commonly known as nabumetone) and 2-(6-methoxy-2-naphthyl)-propionic acid (commonly known as naproxen), via 6-bromo-2-methoxynaphthalene.

As disclosed in British Patent 380,563 (I. G. Farbenindustrie), it is known that 6-bromo-2-naphthol can be prepared by the hydrodebromination of 1,6-dibromo-2-naphthol with an alkali metal sulfite in an aqueous or aqueous-alcoholic solvent. This process is commercially unattractive, however, because of its requiring long reaction times to achieve good yields and because of its generating too much waste.

It would be desirable to find a more efficient process for preparing 6-bromo-2-naphthol and its derivatives from 1,6-dibromo-2-naphthol without the generation of unacceptable amounts of non-recyclable waste and without the use of costly materials.

### Summary of Invention

It has now been found that 1,6-dibromo-2-naphthol can be efficiently hydrodebrominated to 6-bromo-2-naphthol by reacting it with sulfur dioxide in the presence of a base. Thus, the invention resides in a process which comprises reacting 1,6-dibromo-2-naphthol with sulfur dioxide in the presence of a base to prepare 6-bromo-2-naphthol and subsequently, if desired, converting the 6-bromo-2-naphthol product to a 6-bromo-2-alkoxynaphthlene which may then be converted to a pharmaceutically-active derivative, such as nabumetone or naproxen. Embodiments of this invention include:
(1) a process for preparing 6-bromo-2-naphthol by reacting 1,6-dibromo-2-naphthol with sulfur dioxide in the presence of a base,
(2) a process for preparing a 6-bromo-2-alkoxynaphthalene from 6-bromo-2-naphthol which has been produced by reacting 1,6-dibromo-2-naphthol with sulfur dioxide in the presence of a base, and
(3) processes for preparing nabumetone or naproxen from a 6-bromo-2-methoxynaphthalene obtained from 6-bromo-2-naphthol which has been produced by reacting 1,6-dibromo-2-naphthol with sulfur dioxide in the presence of a base.

### Detailed Description

The base employed in the practice of the invention may be any base capable of reacting with the 1,6-dibromo-2-naphthol to form a salt which is then reduced with the sulfur dioxide. However, it is preferably an alkali metal or alkaline earth metal hydroxide, carbonate, alkoxide, or amide, more preferably a sodium or potassium hydroxide, carbonate, alkoxide, or amide, and most preferably potassium carbonate or hydroxide. The amount employed should be at least a stoichiometric amount and is preferably a stoichiometric excess, e.g., an amount such as to provide a base/1,6-dibromo-2-naphthol mol ratio in the range of 2-5/1.

The sulfur dioxide is also preferably used in a superstoichiometric amount, usually an amount such as to provide a sulfur dioxide/1,6-dibromo-2-naphthol mol ratio in the range of 1.2-3.0/1.

As is customary in such reactions, the novel hydrodebromination reaction is conducted in a suitable solvent; and the solvent employed in this hydrodebromination is preferably water or a mixture of water with an alcohol. The alcohol, when employed, is usually a lower alkanol containing 1-4 carbons and is preferably methanol, and it can be used in amounts such as to provide an alcohol/water mol ratio in the range of 0-1/1. Higher amounts of alcohol might also be utilizable, but they would increase the cost of the process.

The hydrodebromination process may be conducted at any temperature which permits the reaction to occur but is preferably conducted at a temperature in the range of 40-70°C. Such temperatures permit the reaction to be completed in a short time, e.g., 1-2 hours, without leading to the by-product formation that may occur at higher temperatures.

The manner in which the ingredients of the reaction mixture are combined does not appear to be absolutely critical. However, to avoid side-reactions that might prevent the attainment of high yields of the desired product, it is usually preferred to combine the other ingredients before adding the 1,6-dibromo-2-naphthol.

After completion of the hydrodebromination reaction, the reaction mixture containing the 6-bromo-2-naphthol product can be worked up in any conventional manner to remove impurities and any solvent that the practitioner wishes to separate from the product. The inorganic waste generated by the process essentially consists of non-toxic bromide salt and sulfate or sulfite, both of which can be recycled if desired.

Derivatives of the 6-bromo-2-naphthol product of the foregoing process can be prepared by any processes suitable for preparing those derivatives from 6-bromo-2-naphthol ― including both processes conducted in the solvent employed in the production of the 6-bromo-2-naphthol and processes conducted in other solvents. Such processes, of course, are already well known. For example:
(1) When it is desired to convert the 6-bromo-2-naphthol to a 6-bromo-2-alkoxynaphthalene, the 6-bromo-2-naphthol may be reacted with an alkylating agent in (a) the water or water-alcohol solvent mixture in which it was synthesized, (b) a solvent mixture obtained by adding a different solvent (e.g., a hydrocarbon, halogenated hydrocarbon, alcohol, or nitrile, such as toluene, ethylene dichloride, isopropyl alcohol, or acetonitrile) to the 6-bromo-2-naphthol solution produced by the hydrodebromination reaction, or (c) ― in cases where the 6-bromo-2-naphthol has been isolated from its synthesis mixture ― one or more of such different hydrocarbon, halogenated hydrocarbon, alcohol, or nitrile solvents, alone or in admixture with water. The alkylating agent used in this reaction, as is already known, is usually a methylating or ethylating agent, most commonly a methylating agent; and it may be, e.g., dimethyl or diethyl sulfate, methanol or ethanol, or an alkyl halide, such as methyl or ethyl chloride.
(2) When it is wished to convert the 6-bromo-2-naphthol to nabumetone, the 6-bromo-2-naphthol is first converted to a 6-bromo-2-methoxynaphthalene intermediate as described above, and the intermediate is then converted to nabumetone by any of the techniques already known for that conversion. For example, as described in U.S. Patent 5,225,603 (Aslam et al.), the 6-bromo-2-methoxynaphthalene may be (a) reacted with N,N-dimethylformamide to form 6-methoxy-2-naphthaldehyde, which is then condensed with acetone to prepare a 4-(6-methoxy-2-naphthyl)-3-buten-2-one intermediate that is then catalytically hydrogenated to nabumetone or (b) reacted with methyl vinyl ketone to form the 4-(6-methoxy-2-naphthyl)-3-buten-2-one intermediate that is then catalytically hydrogenated to nabumetone.
(3) When it is desired to convert the 6-bromo-2-naphthol to naproxen, the 6-bromo-2-naphthol is first converted to a 6-bromo-2-methoxynaphthalene intermediate as described above, and the intermediate is then converted to naproxen by any of the techniques already known for that conversion. For example, as disclosed in U.S. Patent 5,536,870 (Wu), the 6-bromo-2-methoxynaphthalene may be reacted with ethylene to form 2-methoxy-6-vinylnapthalene, which is then carbonylated to racemic 2-(6-methoxy-2-naphthyl)propionic acid, which can be subsequently resolved to isolate the desired naproxen enantiomer.

Both U.S. 5,225,603 and U.S. 4,536,870 are incorporated herein by reference as if fully set forth.

The invention is advantageous in that it provides an efficient process for preparing 6-bromo-2-naphthol and its derivatives from 1,6-dibromo-2-naphthol without the generation of unacceptable amounts of non-recyclable waste and without the use of costly materials..

The following examples are given to illustrate the invention and are not intended as a limitation thereof.

### EXAMPLE 1

Dissolve 3.4g (0.03 mol) of potassium hydroxide pellets in 25g (1.4 mols) of water, slowly introduce 0.8g (0.0125 mol) of sulfur dioxide, then add 3.0g (0.01 mol) of 1,6-dibromo-2-naphthol, and heat the reaction mixture at 45 °C. Analysis of the reaction mixture after one hour shows 99% conversion to 6-bromo-2-naphthol.

### EXAMPLE 2

Dissolve 3.0g (0.01 mol) of 1,6-dibromo-2-naphthol and 1.4g (0.01 mol) of potassium carbonate in a mixture of 29.9g (1.66 mols) of water and 48.6g (1.52 mols) of methanol at room temperature. Introduce 3.3g (0.05 mol) of sulfur dioxide into the resultant clear solution to form a milky mixture and then heat the mixture to 45-50°C to form a clear solution. After two hours at 45-50°C, no reaction has occurred. Add another 2.8g (0.02 mol) of potassium carbonate to effect an exothermic reaction that raises the temperature to 68°C. Maintain the temperature at 65°C for 1.5 hours while monitoring the reaction. GC analysis shows 84% conversion to 6-bromo-2-naphthol after 30 minutes at 65°C and complete conversion after 1.5 hours.

## Claims

1. A process which comprises dehydrobrominating 1,6-dibromo-2-naphthol to prepare 6-bromo-2-naphthol and optionally then converting the 6-bromo-2-naphthol to a derivative, characterized in that the dehydrobromination is conducted by reacting 1,6-dibromo-2-naphthol with sulfur dioxide in the presence of a base.

2. The process of claim 1 which is a process for preparing 6-bromo-2-naphthol.

3. The process of claim 1 which is a process for preparing a 6-bromo-2-alkoxynaphthalene by reacting 1,6-dibromo-2-naphthol with sulfur dioxide in the presence of a base to form 6-bromo-2-naphthol and then alkylating the 6-bromo-2-naphthol.

4. The process of claim 1 which is a process for preparing 4-(6-methoxy-2-naphthyl)-2-butanone by reacting 1,6-dibromo-2-naphthol with sulfur dioxide in the presence of a base to form 6-bromo-2-naphthol, methylating the 6-bromo-2-naphthol to 6-bromo-2-methoxynaphthalene, and then converting the 6-bromo-2-methoxynaphthalene thus obtained to 4-(6-methoxy-2-naphthyl)-2-butanone.

5. The process of claim 1 which is a process for preparing 2-(6-methoxy-2-naphthyl)propionic acid by reacting 1,6-dibromo-2-naphthol with sulfur dioxide in the presence of a base to form 6-bromo-2-naphthol, methylating the 6-bromo-2-naphthol to 6-bromo-2-methoxynaphthalene, and then converting the 6-bromo-2-methoxynaphthalene thus obtained to 2-(6-methoxy-2-naphthyl)propionic acid.

6. A process according to claim 1 wherein the derivative is 6-bromo-2-alkoxynaphthalene obtained by alkylating 6-bromo-2-naphthol.

7. A process according to claim 6 wherein 6-bromo-2-methoxy-naphthalene is further converted to the derivative 4-(6-methoxy-2-naphthyl)-2-butanone.

8. A process according to claim 6 wherein 6-bromo-2-methoxy-naphthalene is further converted to the derivative 2-(6-methoxy-2-naphthyl)propionic acid.

9. The process of any of claims 1-8 wherein the hydrodebromination reaction is conducted in water or a water-alcohol mixture as the solvent and in the presence of a potassium base.

10. The process of any of the preceding claims wherein the hydrodebromination reaction is conducted at a temperature in the range of 40-70°C.

## Patentansprüche

1. Verfahren, das eine Dehydrobromierung von 1,6-Dibrom-2-naphthol, um 6-Brom-2-naphthol herzustellen, und gegebenenfalls dann eine Umwandlung des 6-Brom-2-naphthols in ein Derivat umfasst, dadurch gekennzeichnet, dass die Dehydrobromierung durchgeführt wird, indem 1,6-Dibrom-2-naphthol mit Schwefeldioxid in Gegenwart einer Base umgesetzt wird.

2. Verfahren nach Anspruch 1, das ein Verfahren zur Herstellung von 6-Brom-2-naphthol ist.

3. Verfahren nach Anspruch 1, das ein Verfahren zur Herstellung eines 6-Brom-2-alkoxynaphthalins ist, bei dem 1,6-Dibrom-1-naphthol mit Schwefeldioxid in Gegenwart einer Base umgesetzt wird, um 6-Brom-2-naphthol zu bilden, und dann das 6-Brom-2-naphthol alkyliert wird.

4. Verfahren nach Anspruch 1, das ein Verfahren zur Herstellung von 4-(6-Methoxy-2-naphthyl)-2-butanon ist, bei dem 1,6-Dibrom-2-naphthol mit Schwefeldioxid in Gegenwart einer Base umgesetzt wird, um 6-Brom-2-naphthol zu bilden, das 6-Brom-2-naphthol methyliert wird, um 6-Brom-2-methoxynaphthalin zu bilden, und dann das so erhaltene 6-Brom-2-methoxynaphthin in 4-(6-Methoxy-2-naphthyl)-2-butanon umgewandelt wird.

5. Verfahren nach Anspruch 1, das ein Verfahren zur Herstellung von 2-(6-Methoxy-2-naphthyl)propionsäure ist, bei dem 1,6-Dibrom-2-naphthol mit Schwefeldioxid in Gegenwart einer Base umgesetzt wird, um 6-Brom-2-naphthol zu bilden, das 6-Brom-2-naphthol methyliert wird, um 6-Brom-2-methoxynaphthalin zu bilden, und dann das so erhaltene 6-Brom-2-methoxynaphthalin in 2-(6-Methoxy-2-naphthyl)propionsäure umgewandelt wird.

6. Verfahren nach Anspruch 1, bei dem das Derivat 6-Brom-2-alkoxynaphthalin ist, das durch Alkylierung von 6-Brom-2-naphthol erhalten worden ist.

7. Verfahren nach Anspruch 6, bei dem 6-Brom-2-methoxynaphthalin weiter in das Derivat 4-(6-Methoxy-2-naphthyl)-2-butanon umgewandelt wird.

8. Verfahren nach Anspruch 6, bei dem 6-Brom-2-methoxynaphthalin weiter in das Derivat 2-(6-Methoxy-2-naphthyl)propionsäure umgewandelt wird.

9. Verfahren nach einem der Ansprüche 1 bis 8, bei dem die Hydrodebromierungsreaktion in Wasser oder einer Wasser/Alkohol-Mischung als Lösungsmittel und in Gegenwart einer Kaliumbase durchgeführt wird.

10. Verfahren nach einem der vorhergehenden Ansprüche, bei dem die Hydrodebromierungsreaktion bei einer Temperatur im Bereich von 40 bis 70 °C durchgeführt wird.

## Revendications

1. Procédé qui comprend la débromhydratation de 1,6-dibromo-2-naphtol pour préparer du 6-bromo-2-naphtol et ensuite éventuellement la conversion du 6-bromo-2-naphtol en un dérivé, caractérisé en ce que la débromhydratation est mise en oeuvre par réaction de 1,6-dibromo-2-naphtol avec du dioxyde de soufre en présence d'une base.

2. Procédé selon la revendication 1, qui est un procédé pour préparer du 6-bromo-2-naphtol.

3. Procédé selon la revendication 1, qui est un procédé pour préparer un 6-bromo-2-alcoxynaphtalène par réaction de 1,6-dibromo-2-naphtol avec du dioxyde de soufre en présence d'une base pour former du 6-bromo-2-naphtol et ensuite alkylation du 6-bromo-2-naphtol.

4. Procédé selon la revendication 1, qui est un procédé pour préparer de la 4-(6-méthoxy-2-naphtyl)-2-butanone par réaction de 1,6-dibromo-2-naphtol avec du dioxyde de soufre en présence d'une base pour former du 6-bromo-2-naphtol, méthylation du 6-bromo-2-naphtol en 6-bromo-2-méthoxynaphtalène, et ensuite conversion du 6-bromo-2-méthoxynaphtalène ainsi obtenu en 4-(6-méthoxy-2-naphtyl)-2-butanone.

5. Procédé selon la revendication 1, qui est un procédé pour préparer de l'acide 2-(6-méthoxy-2-naphtyl)propionique par réaction de 1,6-dibromo-2-naphtol avec du dioxyde de soufre en présence d'une base pour former ou 6-bromo-2-naphtol, méthylation du 6-bromo-2-naphtol en 6-bromo-2-méthoxynaphtalène, et ensuite conversion du 6-bromo-2-méthoxynaphtalène ainsi obtenu en acide 2-(6-méthoxy-2-naphtyl)propionique.

6. Procédé selon la revendication 1, dans lequel le dérivé est un 6-bromo-2-alcoxynaphtalène obtenu par alkylation de 6-bromo-2-naphtol.

7. Procédé selon la revendication 6, dans lequel le 6-bromo-2-méthoxynaphtalène est en outre converti en le dérivé 4-(6-méthoxy-2-naphtyl)-2-butanone.

8. Procédé selon la revendication 6, dans lequel le 6-bromo-2-méthoxynaphtalène est en outre converti en le dérivé acide 2-(6-méthoxy-2-naphtyl)propionique.

9. Procédé selon l'une quelconque des revendications 1 à 8, dans lequel la réaction de débromhydratation est mise en oeuvre dans de l'eau ou un mélange eau/alcool à titre de solvant et en présence d'une base potassique.

10. Procédé selon l'une quelconque des revendications précédentes, dans lequel la réaction de débromhydratation est mise en oeuvre à une température située dans la plage de 40 à 70°C.
